# EUROPEAN PATENT SPECIFICATION

(11) **EP 4 353 715 B1**
(45) Date of publication and mention of the grant of the patent: **15.04.2026**
(21) Application number: 23832668.0
(22) Date of filing: 30.04.2023
(51) Int. Cl.: C07D 401/12, A61P 35/00, A61P 35/04

(54) **6-SUBSTITUTED CHIRALLY PURE DIFLUOROPIPERIDINE QUINAZOLINE DERIVATIVE AND PREPARATION METHOD THEREFOR**
6-SUBSTITUIERTES CHIRAL REINES DIFLUORPIPERIDINCHINAZOLINDERIVAT UND HERSTELLUNGSVERFAHREN DAFÜR
DÉRIVÉ DE DIFLUOROPIPÉRIDINE QUINAZOLINE SUBSTITUÉ EN POSITION 6 CHIRALEMENT PUR ET SON PROCÉDÉ DE PRÉPARATION

(30) Priority: 21.07.2022 CN 202210859828
(43) Date of publication of application: 17.04.2024
(73) Proprietor: WEISHANG (SHANGHAI) BIO-PHARMACEUTICAL CO., LTD., Shanghai 201100 (CN)
(72) Inventor: ZHONG, Wei, Minhang District Shanghai 201100 (CN)
(74) Representative: Huang, Liwei
(86) International application number: PCT/CN2023/091927
(87) International publication number: WO 2024/016783

(56) References cited:
- EP-A1- 3 438 107
- CN-A- 107 698 523
- CN-A- 108 069 946
- CN-A- 108 503 596
- CN-A- 108 503 596
- CN-A- 110 343 090
- CN-A- 111 377 850
- CN-A- 113 480 471
- CN-A- 114 057 712
- CN-A- 115 260 153
- JP-A- 2000 344 694
- JACQUES LEPAIH ET AL.: "Efforts towards a Noyori reduction of a 3-ﬂuorpiperidin-4-one with dynamic kinetic resolution", TETRAHEDRON LETTERS, vol. 66, 6 January 2021 (2021-01-06), XP086499864, ISSN: 0040-4039, DOI: 10.1016/j.tetlet.2020.152764

## Description

### TECHNICAL FIELD

The present disclosure relates to a 6-substituted chiral pure difluoropiperidine quinazoline derivative and its preparation method, in particular to (R)-6-[(3,3-difluoro-1-substituted piperidin-4-yl)oxy]-nitrogen-(3-ethynyl-2-fluorophenyl)-7-methoxyquinazoline-4-amine (I) and its preparation method.

### BACKGROUND ART

The 6-substituted chiral pure difluoropiperidine quinazoline derivative, a type of molecular targeted anti-tumor drug and a tyrosine kinase inhibitor for epidermal growth factor receptor (EGFR-TKI), is used to treat brain metastases of non-small cell lung cancer (SCLC), meningeal metastases, squamous cell carcinoma of the head and neck, squamous cell carcinoma, brainstem tumors, primary brain cancer, gliomas, lung squamous cell carcinoma, or other types of cancer. China Patent Applications 201610982608.6 (EP3438107A1) and 201810423951.8 (WO20191966 A1) describe the preparation method and application of the above quinazoline derivatives. Different drug manufacturing processes may have significant differences in the quality of drugs produced, thus affecting the efficacy of drugs. Therefore, it is of great significance to develop an optimized reproducible, safe, and impurity-controllable drug manufacturing process with a high yield, and high chiral selectivity for scaling up clinical application and commercialization of drugs and cutting production costs.

CN108503596 A discloses the use of a 2-amino-benzonitrile and a diphenyl formadinine for the preparation of erlotinib.

### SUMMARY

The present disclosure intends to provide a repeatable, safe, impurity-controllable, and low-cost method for the preparation of 6-substituted chiral pure difluoropiperidine quinazoline derivatives, especially the preparation of chiral pure difluoropiperidine intermediates and quinazoline ring closure with higher efficiency, higher yield, and higher chiral selectivity, suitable for large-scale drug production, and to provide qualitative and quantitative information for clinical and commercial production of high-quality and low-cost drugs.

The present disclosure provides a method for preparing 6-substituted chiral pure difluoropiperidine quinazoline derivatives with higher yield, higher chiral selectivity, repeatability, safety, and impurity control. The 6-substituted chiral pure difluoropiperidine quinazoline derivative is represented by Formula (I): 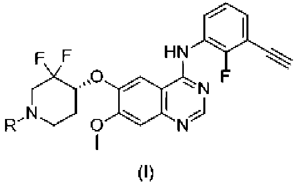 where R is Boc, oxetane, or methyl.

One technical solution adopted by the present disclosure is to provide a safe and repeatable method for preparing chiral pure difluoropiperidine intermediates with higher yield and higher chiral selectivity.

The invention is set out in teh appended set of claims.

The method comprises the following steps: Add triethylamine and formaldehyde into 3,3-difluoropiperidinol derivative 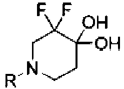 to react in the presence of chiral Ru(II) catalyst and organic solvent until TLC shows that the raw materials disappear; then add base to obtain substituted chiral difluoropiperidinol

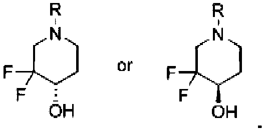

One technical solution adopted by the present disclosure is to provide a high-yield, repeatable ring closure method for preparing quinazoline derivatives (I). The method comprises a quinazoline ring-closure step: The 2-amino-benzonitrile derivative 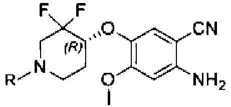 reacts with (E)-N,N'-bisphenylformamidine derivative 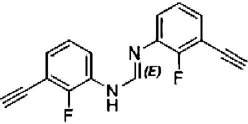 in the presence of an organic solvent and glacial acetic acid to obtain 6-substituted chiral pure difluoropiperidine quinazoline derivatives 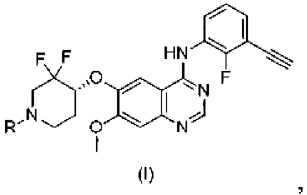 where R is Boc, oxetane or methyl.

### BRIEF DESCRIPTION OF DRAWINGS

The illustration of non-limiting embodiments by the following drawings will better reveal other features, objectives, and advantages of the present disclosure:
FIG. 1 is a schematic diagram of the characteristic synthesis of 6-substituted chiral pure difluoropiperidine quinazoline derivatives;
FIG. 2 is a schematic diagram of the pharmacodynamics of (R)-6-((3,3-difluoro-1-methylpiperidin-4-yl)oxy)-N-(3-ethynyl-2-fluorophenyl)-7-methoxyquinazoline-4-amine in a mouse intracranial PDX model;
FIG. 3 is a comparative schematic diagram of the survival rate of (R)-6-((3,3-difluoro-1-methylpiperidin-4-yl)oxy)-N-(3-ethynyl-2-fluorophenyl)-7-methoxyquinazoline-4-amine in an animal model of intracranial meningeal metastasis in mice;
FIG. 4 is a schematic diagram of the mechanism of action of (R)-6-((3,3,difluoro-1-methylpiperidin-4-yl)oxy)-N-(3-ethynyl-2-fluorophenyl)-7-methoxyquinazoline-4-amine.

### DETAILED DESCRIPTION

The terms involved in the present disclosure are explained as follows:
The term "isomer" used herein refers to different compounds having the same molecular formula. The "stereoisomers" are isomers that differ only in the spatial arrangement of atoms. The term "isomer" as used herein includes any and all geometrical isomers and stereoisomers. For example, it includes cis and trans isomers of geometric double bonds, also referred to as E- and Z-isomers: R- and S-enantiomers: diastereomers, (D) isomers and (L) isomers, racemic mixtures thereof, and other mixtures thereof disclosed herein.

The double bond around the carbon-carbon substituent is designated in either a "Z" or an "E" configuration, where the terms "Z" and "E" are used according to IUPAC standards. Unless otherwise indicated, the structure describing the double bond includes both "E" and "Z" isomers.

The alternative substituent surrounding the carbon-carbon double bond may be "cis" or "trans", where "cis" means that the substituent is on the same side of the double bond while "trans" means that the substituent is on both sides of the double bond. The arrangement of the surrounding carbon rings of the substituent may also be designated as "cis" or "trans". The term "cis" denotes substituents on the same side of the ring plane, and "trans" signifies substituents on both sides of the ring plane. The mixture of compounds in which the substituents are on the same and opposite sides of the plane of two rings is denoted "cis/trans".

The term "enantiomer" used herein is a pair of stereoisomers that are non-overlapping mirror images of each other. A mixture of a pair of enantiomers in any ratio may be referred to as a "racemic" mixture. The symbol "(±)" is used to specify racemic mixtures as appropriate. "Diastereoisomerism" refers to a pair of stereoisomers that have at least two asymmetric atoms, but are not mirror images of each other. Absolute stereochemistry is specified according to the Cahn-Ingold-Prelog R-S system. When a compound is enantiomeric, its stereochemistry in each chiral carbon can be designated as R or S. A resolved compound whose absolute configuration is unknown can be designated as (+) or (-), depending on the direction (right-handed or left-handed) in which it rotates the plane polarized light at the wavelength of the sodium D-line.

In the absolute configuration, for a compound with each asymmetric atom as (R)- or (S)-, the pharmaceutical composition and method include all such possible isomers, including racemic mixtures, optically pure forms and a mixture of intermediates. The optically active (R)- and (S)- can also be prepared using chiral synthesis methods or chiral reagents, or resolved using conventional techniques.

A composition of the term "enantiomeric excess" or "% enantiomeric excess" as used herein can be calculated using the formula shown below. In the examples shown below, the composition contains 90% of one enantiomer, for example, the S enantiomer, and contains 10% of the other enantiomer, for example, the R enantiomer.

The ee value = (90-10) / 100 = 80%.

Thus, a composition containing 90% of one enantiomer and 10% of the other enantiomer is said to have an enantiomeric excess of 80%. Some of the compositions described herein contain at least about 50% enantiomeric excess, about 75%, about 90%, about 95%, or about 99% of the S enantiomer. In other words, the composition comprises an enantiomeric excess of the S enantiomer in the R enantiomer. In other embodiments, some of the compositions described herein contain at least about 50% enantiomeric excess, about 75%, about 90%, about 95%, or about 99% of the R enantiomer. In other words, the composition comprises an enantiomeric excess of the R enantiomer in the S enantiomer. For example, one isomer/enantiomer may, in some embodiments, provide the ee value of the corresponding enantiomer and may also be referred to as "optical enrichment", or "enantiomeric enrichment". "Enantiomerically pure" and "non-racemic" are used interchangeably herein. These terms mean that the weight percent of one of the enantiomers in a composition is greater than the amount of the control mixture of the racemic composition in one enantiomer (e.g., a weight ratio greater than 1:1). For example, the enantiomerically enriched (by weight) S enantiomer of the S enantiomer relative to the R enantiomer, such as at least about 75% by weight of the compound having greater than about 50%, is also at least about 80% by weight. In some embodiments, the enrichment is greater than about 80% by weight to provide a preparation of "substantially enantiomerically enriched", "substantially enantiomerically pure" or "substantially non-racemic", which means that its enantiomer has at least 85% composition by weight relative to one of the other enantiomers, such as at least about 90% formulation by weight and further, for example, at least about 95% by weight. In certain embodiments, the compounds provided herein can be from about 90% of at least one enantiomer by weight. In other embodiments, the compound can be from at least about 95%, about 98%, or about 99% by weight of one enantiomer. In some embodiments, the compound is an (S)- and (R)-racemic mixture. In other embodiments, provided herein is a method wherein a single compound (S)- of the mixture primarily a mixture of compounds present or (R)- is a mixture of primarily present compounds. For example, the compound mixture has an (S)-enantiomeric excess of greater than about 55%, about 60%, about 65%, about 70%, about 75%, about 80%, about 85%, about 90%, about 95%, about 96%, about 97%, about 98%, about 99%, about 99.5%, or more. In other embodiments, the mixture of compounds has an (S)-enantiomeric excess of greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5%, or higher. In other embodiments, the (R)-enantiomer purity of the compound mixture is greater than about 55%, about 60%, about 65%, about 70%, about 75%, about 80%, about 85%, about 90%, about 95%, about 96%, about 97%, about 98%, about 99%, about 99.5% or more. In other embodiments, the mixture of compounds has an (R)-enantiomeric excess of greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than 95% to about 99.5%, greater than about 96% to about 99.5%, greater than about 97% to about 99.5%, greater than about 98% to greater than about 99.5%, greater than about 99% to about 99.5%, or more.

In other embodiments, the compound mixture comprises, in addition to their stereochemical orientation, i.e. (S)- or (R)- identical chemical entity. For example, if a compound has a -CH(R)-unit disclosed herein, and R is not hydrogen, then -CH(R)- is the orientation of each identical chemical entity in the (S)- or (R)-stereochemistry. In some embodiments, the (S)-isomer in a mixture of identical chemical entities is present in an (S)-enantiomeric excess of greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5%, or more.

In another embodiment, the (R)-isomer is in a mixture of identical chemical entities (except for their stereochemical orientation) relative to the (S)isomer, at about 55%, about 60%, about 65%, about 70%, about 75%, about 80%, about 85%, about 90%, about 95%, about 96%, about 97%, about 98%, about 99%, about 99.5% or higher. In some embodiments, the (R)-isomer in a mixture of identical chemical entities (except for their stereochemical orientation) is present in the (R)-enantiomeric excess of greater than about 55% to about 99.5%, greater than about 60% to about 99.5%, greater than 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5%, or more.

The enantiomers can be separated from the racemic mixture by any of the methods known to those skilled in the art, including chiral high performance liquid chromatography (HPLC), and formation and crystallization of chiral salts. See, for example, Enantiomers, Racemates and Resolutions (Jacques, Ed., Wiley Interscience, New York, 1981); Wilen et al., Tetrahedron 33:2725 (1977); Stereochemistry of Carbon Compounds (E. L. Eliel, Ed., McGraw-Hill, NY, 1962); and Tables of Resolving Agents and Optical Resolutions p. 268 (E. L. EIM, Et al. , Univ. of Notre Dame Press, Notre Dame, Ind. 1972).

Optical isomers can also be obtained by resolution of the racemic mixture with an optically active acid or base in a conventional manner, for example, by formation of diastereomeric salts. Examples of suitable acids include, but are not limited to, tartaric acid, diacetyl, dibenzoyl, xylyl tartaric acid, and camphorsulfonic acid. Diastereomeric crystallization is followed by separation of the isomers from a mixture of optically active bases of these salts. Alternatively, the reaction of a disclosed compound with an optically pure acid or optically pure isocyanate in an activated form involves the synthesis of a covalent diastereomeric molecule. The synthetic enantiomers can be separated by conventional methods such as chromatography, distillation, crystallization or sublimation followed by hydrolysis to provide enantiomerically enriched compounds. Optically active compounds can also be obtained by using active raw materials. In some embodiments, these isomers may be in the form of free acids, free bases, esters or salts.

The term "CDCl₃" refers to deuterated chloroform.

The term "DMSO-d6" refers to deuterated dimethyl sulfoxide

The term "LC-MS: (ESI)" refers to electrospray ionization liquid chromatography-mass spectrometry

The present disclosure provides a preparation method for 6-substituted chiral pure difluoropiperidine quinazoline derivatives as shown in Formula (I); 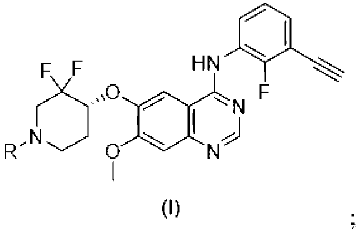 wherein R is Boc, oxetane, or methyl.

The preparation process of the 6-substituted chiral pure difluoropiperidine quinazoline derivatives described in the present disclosure can be easily carried out by those skilled in the art of synthetic chemistry. For example, related methods and intermediates disclosed herein.

In some embodiments, the concentration of one or more compounds as described herein can be greater than about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50% , about 4.25%, about 4%, about 3.75%, about 3.50%, about 3.25%, about 3%, about 2.75%, about 2.50%, about 2.25%, about 2%, about 1.75%, about 1.50%, about 1.25%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001% weight/weight ratio, weight/volume ratio, or volume/volume ratio. In some embodiments, the concentration of one or more compounds described herein can range from about 0.0001% to about 50%, from about 0.001% to about 40%, from about 0.01% to about 30%, from about 0.02% to about 29%, from about 0.03% to about 28%, from about 0.04% to about 27%, from about 0.05% to about 26%, from about 0.06% to about 25%, from about 0.07% to about 24%, from about 0.08% to about 23%, from about 0.09% to about 22%, from about 0.1% to about 21%, from about 0.2% to about 20%, from about 0.3% to about 19%, from about 0.4% to about 18%, from about 0.5% to about 17%, from about 0.6% to about 16%, from about 0.7% to about 15%, from about 0.8% to about 14%, from about 0.9% to about 12%, from about 1% to about 10% weight/weight ratio, weight/volume ratio, or volume/volume ratio. In some embodiments, the concentration of one or more compounds as disclosed herein can range from about 0.001% to about 10%, from about 0.01% to about 5%, from about 0.02% to about 4.5%, from about 0.03% to about 4%, from about 0.04% to about 3.5%, from about 0.05% to about 3%, from about 0.06% to about 2.5%, from about 0.07% to about 2%, from about 0.08% to about 1.5%, from about 0.09% to about 1%, from about 0.1% to about 0.9% weight/weight ratio, weight/volume ratio or volume/volume ratio.

The enantiomer purity of the preparation method of the 6-substituted chiral pure difluoropiperidine quinazoline derivatives described in the present disclosure is greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5%, or higher.

The preparation of 6-substituted chiral pure difluoropiperidine quinazoline derivatives described herein may use protocols known in the art, including, for example, those described herein. The present disclosure, the manufacturing process of 6-substituted chiral pure difluoropiperidine quinazoline derivatives, demonstrates surprisingly excellent properties (e.g., high efficiency, high yield, high chiral selectivity, repeatability, safety, controllable impurities and low cost), which is applicable for the preparation of large-scale drug production and provides qualitative and quantitative information for clinical and commercial production of high-quality and low-cost drugs.

The present disclosure provides a preparation method for 6-substituted chiral pure difluoropiperidine quinazoline derivatives as shown in Formula (I), which comprises the quinazoline ring-closure step: 2-amino-benzonitrile derivatives 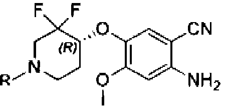 reacted with (E)-N,N'-bisphenylformamidine derivatives 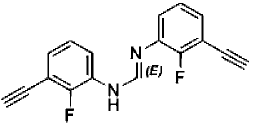 in the presence of organic solvent and glacial acetic acid to obtain 6-substituted chiral pure difluoropiperidine quinazoline derivatives 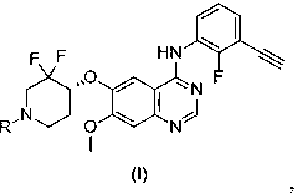 wherein R is Boc, oxetane, or methyl.

The ring-closure method in the present disclosure for preparing quinazoline derivatives (I) improves the ring-closure yield, reduces material loss and production costs, and provides a large batch of drugs with high quality and controllable impurities for clinical and commercial purposes. The high yield, high chiral selectivity, optimized reproducible, safe and impurity-controlled drug manufacturing process of the present disclosure is of great significance for scale-up clinical and commercial purposes of drugs and reduction of production costs.

As one embodiment, said 2-amino-benzonitrile derivative is (R)-4-(4-amino-5-cyano-2-methoxyphenoxy)-3,3-difluoropiperidine-1-carboxylic acid tert-butyl ester.

As one embodiment, said organic solvent is toluene.

As one embodiment, said reaction temperature is 90 - 110°C and reaction time is 2 - 4 h.

As one embodiment, said equivalent ratio of 2-amino-benzonitrile derivative to (E)-N,N'-bisphenylformamidine derivative is 1: 1-1.2.

In one embodiment, the preparation of quinazoline ring closure comprises the steps of: (E)-N,N'-bisphenylformamide derivative (1 - 1.2 equivalents) and glacial acetic acid (1 - 1.2 V) were added to a toluene solution (5 - 10 V) of 2-amino-benzonitrile derivative (1 equivalent), heated at 90 - 110°C and stirred for 2 - 4 h to obtain quinazoline derivatives.

The preparation of 2-amino-benzonitrile derivatives comprises the steps:
S1: The substituted chiral difluoropiperidinol 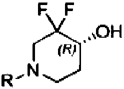 reacts with 5-fluoro-4-methoxy-2-nitrobenzonitrile 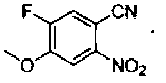 in the presence of potassium tert-butoxide and an organic solvent at 10± 5°C to obtain a 2-nitro-benzonitrile derivative 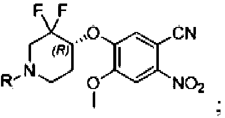
S2: 2-nitro-benzonitrile derivatives 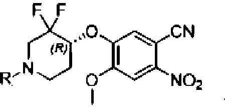 were reduced with sodium dithionite and organic solvent at room temperature to obtain 2-amino-benzonitrile derivatives

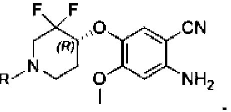

As one embodiment, the organic solvent in step S1 comprises tetrahydrofuran. The organic solvent in step S2 comprises ethanol.

As one embodiment, the reaction time in step S1 is 2 - 4 h.

As one embodiment, when the structural formula of the 2-nitro-benzonitrile derivative in step S1 is 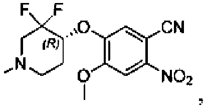 step S1 comprises:
S1-1: Substituted chiral difluoropiperidinol 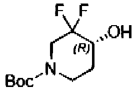 (e.g., 1 equivalent) reacted with 5-fluoro-4-methoxy-2-nitrobenzonitrile 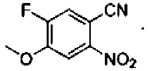 (0.8 - 1.2 equivalents) in the presence of potassium tert-butoxide (1.2 - 2.0 equivalents) and organic solvent at 10 ± 5°C to obtain chiral pure (R)-4-(5-cyano-2-methoxy-4-nitrophenoxy)-3,3-difluoropiperidine-1-carboxylic acid tert-butyl ester 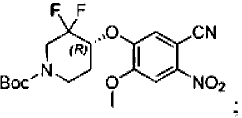
S1-2: Under nitrogen protection, chiral pure (R)-4-(5-cyano-2-methoxy-4-nitrophenoxy)-3,3-difluoropiperidine-1-carboxylic acid tert-butyl ester reacted in the presence of hydrochloric acid/ethyl acetate solution to obtain (R)-5-((3,3-difluoropiperidine-4-yl)oxy)-4-methoxy-2-nitrobenzonitrile 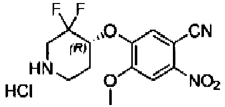 at 25 ± 5°C;
S1-3: Under nitrogen protection, (R)-5-((3,3-difluoropiperidine-4-yl)oxy)-4-methoxy-2-nitrobenzonitrile (1 equivalent) reacted in the presence of organic solvent (5 - 20 V), formaldehyde aqueous solution (4 - 6 V) and NaBH(OAc)₃ (2.5 - 3.5 equivalents) to obtain 2-nitro-benzonitrile derivatives

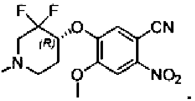

As one embodiment, the organic solvent in step S1-1 comprises tetrahydrofuran, and the organic solvent in step S1-3 comprises methanol. In step S1-3, the preferred reaction temperature is room temperature and the reaction time is 1 - 3 h; the preferred equivalent ratio of compound 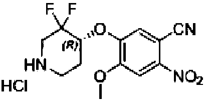 to NaBH(OAc)₃ is 1:2 - 1:4.

As one embodiment, in step S2, sodium dithionite was added to the compound 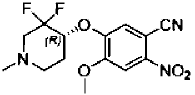 and stirred, then concentrated hydrochloric acid was dropped and stirred continuously to obtain a compound 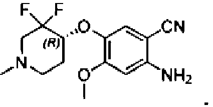 The preferred stirring time is 2 - 4 h; the continuous stirring time is 2 - 4 h; and the reaction temperature is room temperature. The preferred equivalent ratio of the compound 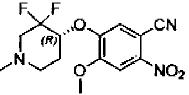 to sodium dithionite and concentrated hydrochloric acid is 1: (2.5 - 4.5): (5 - 7.5).

As one embodiment, said preparation of substituted chiral difluoropiperidinol comprises the steps:
3,3-difluoropiperidinol derivatives 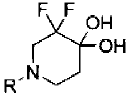 reacted with triethylamine and formaldehyde added in the presence of chiral Ru(II) catalyst and organic solvent until TLC showed that the raw material disappeared to obtain substituted chiral difluoropiperidinol 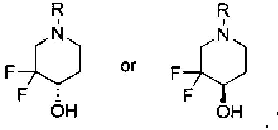 The preparation method of chiral pure difluoropiperidine intermediate described in the present disclosure is characterized by high yield, high chiral selectivity, avoiding enantiomeric waste and artificial material waste caused by chiral resolution to reduce costs; avoiding direct use of hydrogen reaction to reduce production risks and improve safety.

As one embodiment, said 3,3-difluoropiperidinol derivative is 3,3-difluoro-1-methylpiperidin-4-keto or 3,3-difluoro-4-oxopiperidin-1-carboxylic acid tert-butyl ester.

Preferably, the 3,3-difluoropiperidinol derivative is 3,3-difluoro-4-oxopiperidin-1-carboxylic acid tert-butyl ester.

As one embodiment, said chiral Ru(II) catalyst is (*R*)-RuCl[(p-cymene(BINAP)]Cl or RuCl[(*R*,*R*-TsDPEN)](p-cymene) to obtain 3,3-difluoropiperidinol derivative 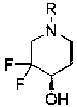 with chirality of R.

Preferably, the chiral Ru(II) catalyst is RuCl[(*R,R*-TsDPEN)](p-cymene).

As one embodiment, said chiral Ru(II) catalyst is (*S*)-RuCl[(p-cymene(BINAP)]Cl or RuCl[(*S,S*-TsDPEN)](p-cymene) to obtain 3,3-difluoropiperidinol derivatives 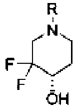 with chirality of S.

Preferably, said chiral Ru(II) catalyst is RuCl[(*S*,*S*-TsDPEN)](p-cymene).

As one embodiment, said organic solvent in the preparation of substituted chiral difluoropiperidinol is DMF, DME or tetrahydrofuran. Preferably, tetrahydrofuran.

As one embodiment, said base in the preparation of substituted chiral difluoropiperidinol is potassium tert-butoxide.

As one embodiment, said equivalent ratio of 3,3-difluoropiperidinol derivative, triethylamine, formaldehyde and base in the preparation of substituted chiral difluoropiperidinol is 1: (1.5 - 2): (1 - 1.5): (1 - 2).

In one embodiment, the preparation of chiral pure difluoropiperidine intermediate comprises the steps:
Organic solvent (3 - 5 V) of 3,3-difluoropiperidinol derivatives (1 equivalent) reacted with chiral Ru(II) catalyst (0.5% - 1%) catalyst with triethylamine (1.5 - 2 equivalents) and formaldehyde (1 - 1.5 equivalents) added until the raw material disappeared, and reacted with base (1 - 2 equivalents) added to obtain chiral pure product.

The present disclosure is described in detail as follows with reference to specific embodiments. The following embodiments will help provide further understanding of the present disclosure for those skilled in the art, and not in any way limit the present disclosure.

### Embodiment 1 Preparation Method of Substituted Chiral Pure Difluoropiperidinol with High Yield and High Chiral Selectivity

### 1.1 Preparation method of substituted chiral pure difluoroperidinol

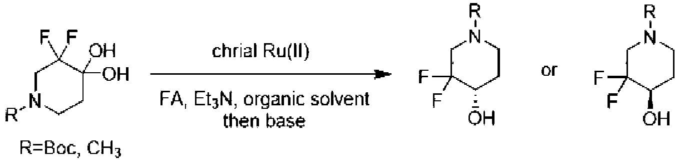

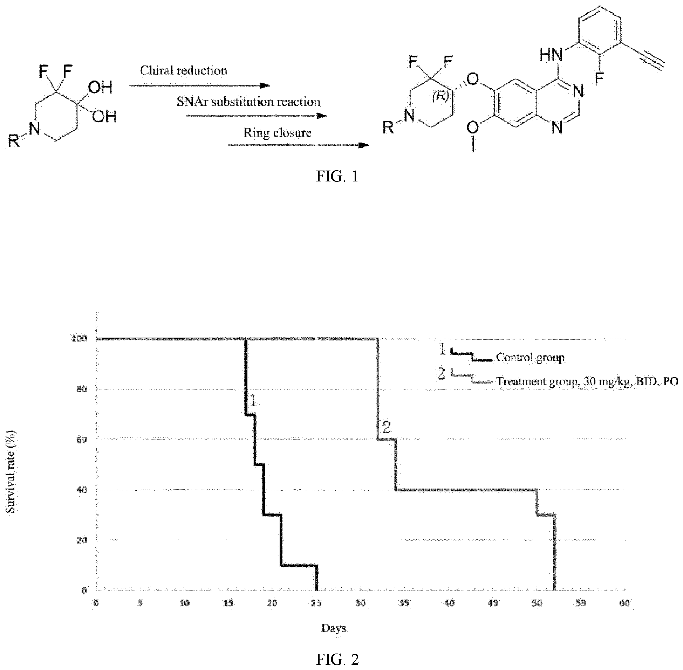

Correspond to the chiral reduction step in FIG. 1; the preparation method is as follows:
Organic solvent (3 - 5 V) of 3,3-difluoropiperidinol derivatives (1 equivalent) reacted with chiral Ru(II) catalyst (0.5% - 1%) catalyst with triethylamine (1.5 - 2 equivalents) and formaldehyde (1 - 1.5 equivalents) added until the raw material disappeared, and reacted with base (1 - 2 equivalents) added to obtain chiral pure product.

Said organic solvent is DMF, DME or tetrahydrofuran; preferably, tetrahydrofuran, 5 V.

Said 3,3-difluoropiperidinol derivative is 3,3-difluoro-1-methylpiperidin-4-keto or 3,3-difluoro-4-oxopiperidin-1-carboxylic acid tert-butyl ester, preferably, 3,3-difluoro-4-oxopiperidin-1-carboxylic acid tert-butyl ester.

Said chiral Ru(II) catalyst is (*R*)-RuCl[(p-cymene(BINAP)]Cl or RuCl[(*R*,*R*-TsDPEN)](p-cymene) to obtain 3,3-difluoropiperidinol derivatives with the chirality of R, preferably, 1% RuCl[(*R,R*-TsDPEN)](p-cymene).

Said chiral Ru(II) catalyst is (*S*)-RuCl[(p-cymene(BINAP)]Cl or RuCl[(*S*,*S*-TsDPEN)](p-cymene) to obtain 3,3-difluoropiperidinol derivatives with the chirality of S, preferably, 1% RuCl[(*S,S*-TsDPEN)](p-cymene).

Said base is potassium tert-butoxide.

In this embodiment, 8 groups of preparation schemes for substituted chiral pure difluoropiperidinol are provided. The involved reactants, reagent conditions and yield are shown in Table 1:

**Table 1**

| Group | Reactant (1 equivalent) | Reagent condition | Reaction main product | Yield | %ee |
|---|---|---|---|---|---|
| 1 | 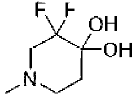 | RuCl[(S,S-TsDPEN)](p-cymene) (0.5 - 1%), organic solvent (3 - 5 V), triethylamine (1.5 - 2 equivalents) and formaldehyde (1 - 1.5 equivalents), potassium tert-butoxide (1 - 2 equivalents) | 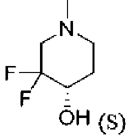 | >70% | >50% |
| 2 | 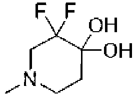 | (*S*)-RuCl[(p-cymene(BINAP)]Cl (0.5 - 1%), organic solvent (3 - 5 V), triethylamine (1.5 - 2 equivalents) and formaldehyde (1 - 1.5 equivalents), potassium tert-butoxide (1 - 2 equivalents) | 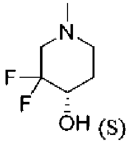 | >70% | >50% |
| 3 | 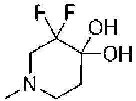 | RuCl[(R,R-TsDPEN)](p-cymene) (0.5 - 1%), organic solvent (3 - 5 V), triethylamine (1.5 - 2 equivalents) and formaldehyde (1 - 1.5 equivalents), potassium tert-butoxide (1 - 2 equivalents) | 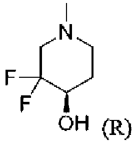 | >70% | >50% |
| 4 | 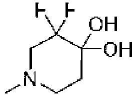 | (R)-RuCl[(p-cymene(BINAP)]Cl (0.5 - 1%) organic solvent (3 - 5 V), triethylamine (1.5 - 2 equivalents) and formaldehyde (1 - 1.5 equivalents), potassium tert-butoxide (1 - 2 equivalents) | 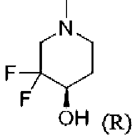 | >70% | >90% |
| 5 | 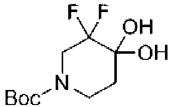 | RuCl[(S,S-TsDPEN)](p-cymene) (0.5 - 1%), organic solvent (3 - 5 V), triethylamine (1.5 - 2 equivalents) and formaldehyde (1 - 1.5 equivalents), potassium tert-butoxide (1 - 2 equivalents) | 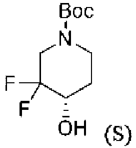 | >70% | >90% |
| 6 | 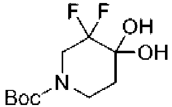 | (*S*)-RuCl[(p-cymene(BINAP)]Cl (0.5 - 1%), organic solvent (3 - 5 V), triethylamine (1.5 - 2 equivalents) and formaldehyde (1 - 1.5 equivalents), potassium tert-butoxide (1 - 2 equivalents) | 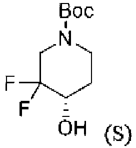 | >70% | >90% |
| 7 | 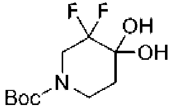 | RuCl[(R,R-TsDPEN)](p-cymene) (0.5 - 1%), organic solvent (3 - 5 V), triethylamine (1.5 - 2 equivalents) and formaldehyde (1 - 1.5 equivalents), potassium tert-butoxide (1 - 2 equivalents) | 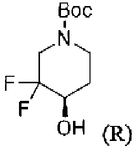 | >70% | >90% |
| 8 | 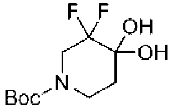 | (R)-RuCl[(p-cymene(BINAP)]Cl (0.5 - 1%) organic solvent (3 - 5 V), triethylamine (1.5 - 2 equivalents) and formaldehyde (1 - 1.5 equivalents), potassium tert-butoxide (1 - 2 equivalents) | 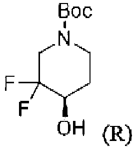 | >70% | >90% |

### 1.2 Specifically, preparation of (R)-3,3-difluoro-4-hydroxypiperidine-1-carboxylic acid tert-butyl ester with high chiral selectivity

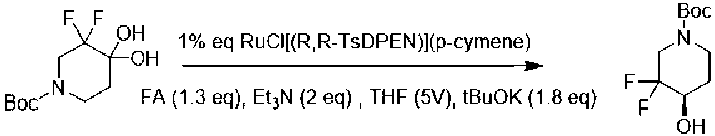

Detailed steps: 3,3-difluoro-4,4-2-hydroxypiperidine-1-carboxylic acid tert-butyl ester (5 kg, 1 equivalent), Ru(II) catalyst RuCl[(R,R-TsDPEN)](p-cymene) (135 g, 1% equivalent) and tetrahydrofuran solution (16.2 L, 5 V) were added to a nitrogen blanketed reactor and stirred until completely dissolved. Triethylamine (4.3 kg, 2 equivalents) was added at room temperature and formaldehyde (1.27 kg, 1.3 equivalents) was added dropwise, and stirred for 2 h at 35 ± 5°C. TLC showed that the raw material disappeared completely. Potassium tert-butoxide (4.29 kg, 1.8 equivalents) was added and stirred at 10 ± 5°C for 2 h before cooling to 5 ± 5°C. Water (32.4 L, 10 V) was added, stirred to precipitate a solid, filtered and dried to obtain a white solid of chiral pure (R)-3,3-difluoro-4-hydroxypiperidine-1-carboxylic acid tert-butyl ester (4.62 kg, yield: 94.7%, %ee > 99%).

### 1.3 Specifically, preparation of enantiomer (S)-3,3-difluoro-4-hydroxypiperidine-1-carboxylic acid tert-butyl ester with high chiral selectivity

Detailed steps: 3,3-difluoro-4,4-2-hydroxypiperidine-1-carboxylic acid tert-butyl ester (50 g, 1 equivalent), Ru(II) catalyst RuCl[(S,S-TsDPEN)](p-cymene) (1.35 g, 1% equivalent) and tetrahydrofuran solution (5 L, 5 V) were added to a nitrogen blanketed reactor and stirred until completely dissolved. Triethylamine (43 g, 2 equivalents) was added at room temperature, formaldehyde (12.7 g, 1.3 equivalents) was added dropwise, and stirred for 2 h at 35 ± 5°C. TLC showed that the raw material disappeared completely. Potassium tert-butoxide (42.9 g, 1.8 equivalents) was added and stirred at 10 ± 5°C for 2 h before cooling to 5 ± 5°C. Water (300 mL, 10 V) was added, stirred to precipitate a solid, filtered and dried to obtain a white solid of chiral pure (S)-3,3-difluoro-4-hydroxypiperidine-1-carboxylic acid tert-butyl ester (yield: 90%, %ee > 98%).

The preparation method of chiral pure difluoropiperidine intermediate described in the present disclosure, especially the preparation of (R)-3,3-difluoro-4-hydroxypiperidine-1-carboxylic acid tert-butyl ester for the manufacturing of final drugs, is characterized by high yield (> 90%), high chiral selectivity (%ee > 98%), avoiding enantiomeric waste and artificial material waste caused by chiral resolution to reduce costs; avoiding direct use of hydrogen reaction to reduce production risks and improve safety.

### Embodiment 2 Preparation of Quinazoline Derivatives (I) from (E)-N,N'-Bisphenylformamidine Derivatives by Acid Catalyzed Ring-closure Method

### 2.1 Preparation of quinazoline derivatives from (E)-N,N'-Bisphenylformamidine Derivatives by acid catalyzed ring-closure method

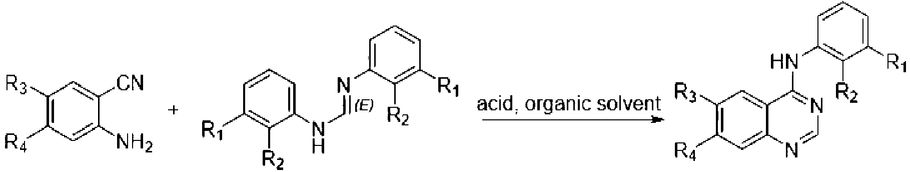

R1 is ethynyl, R2 is F, R3 is substituted difluoropiperidinol and R4 is methoxy;

The acid is glacial acetic acid and said organic solvent is toluene.

Under nitrogen protection, substituted (E)-N,N'-diphenylimine amide (1.1 equivalents) and glacial acetic acid (1 V) were added dropwise at room temperature to a toluene solution of substituted 2-aminobenzonitrile (1 equivalent) and heated to reflux for 2 h until the raw material disappeared and the ring closure was successfully completed to obtain quinazoline derivatives with a yield > 70%.

### 2.2 Specifically, preparation of (R)-6-((3,3-difluoro-1-methylpiperidin-4-yl)oxy)-N-(3-ethynyl-2-fluorophenyl)-7-methoxyquinazoline-4-amine using (E)-N,N'-bis(3-ethynyl-2-fluorophenyl)formamidic acid as a catalyst

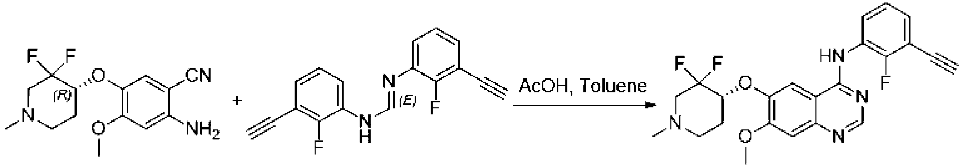

Correspond to the ring-closure steps in FIG. 1; detailed steps: Under nitrogen protection, (E)-N,N'-bis(3-ethynyl-2-fluorophenyl)fomamidine (1.17 kg, 1.1 equivalents) and glacial acetic acid (1 V) were added dropwise to a toluene solution (8 V) of (R)-2-amino-5-((3,3-difluom-1-methylpiperidin-4-yl)oxy)-4-methoxybenzonitrile (1.13 kg, 1 equivalent) at room temperature, heated to 95 ± 5°C and refluxed for 2 h until the raw material disappeared, cooled to 20 ± 5°C, continued to stir for 12 h, and filtered to obtain (R)-6-((3,3-difluoro-1-methylpiperidin-4-yl)oxy)-N-(3-ethynyl-2-fluorophenyl)-7-methoxyquinazoline-4-amine (1.28 kg, yield > 76%).

### 2.3 Specifically, preparation of (R)-4-((4-((3-ethynyl-2-fluorophenyl)amino)-7-methoxyquinazoline-6-yl)oxy)-3,3-difluoropiperidine-1-carboxylic acid tert-butyl ester using (E)-N,N'-bis(3-ethynyl-2-fluorophenyl)formamidic acid as a catalyst

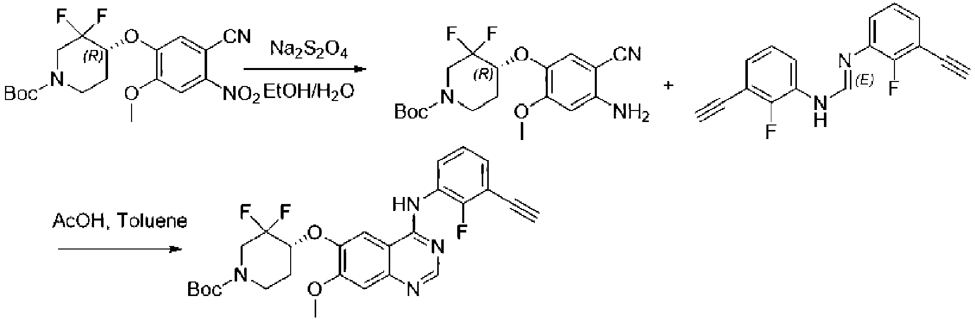

Correspond to the ring-closure steps in FIG. 1; detailed steps: Aqueous solution (200 mL, 8 V) of sodium dithionite (31.6 g, 0.18 mol) was added dropwise to ethanol (200 mL, 8 V) solution of (R)-4-(5-cyano-2-methoxy-4-nitrophenoxy)-3,3-difluoropiperidine-1-carboxylic acid tert-butyl ester (25 g, 0.06 mol) at 25°C. The reaction mixture was stirred at 20°C for 16 h, concentrated and evaporated to 260 mL of solvent at 40°C. Water (50 mL, 2 V) was added to the mixture, and the pH of the mixture was adjusted to 10 - 11 with 25% aqueous sodium hydroxide solution. The mixture was extracted 3 times with methyl tert-butyl ether (50 mL). The organic layers were combined, and dried with anhydrous sodium sulfate. The solid was filtered off and the filtrate was concentrated to obtain a residue slurryed in n-heptane (75 mL, 3 V), and filtered. The filter cake was collected, washed with n-heptane (25 mL, 1 V), and dried under vacuum to obtain a pale yellow solid (9.6 g, 0.025 mol). ¹HNMR: (400 MHz, DMSO) δ ppm: 7.11 (s, 1H), 6.43 (s, 1H), 5.82 (s, 2H), 4.54 - 4.34 (m, 1H), 3.92 - 3.78 (m, 1H), 3.77(s, 3H), 3.66 (m, 1H), 3.61 - 3.51 (m, 1H), 3.30(m, 1H), 1.94 - 1.82 (m, 1H), 1.76 (m, 1H), 1.43 (s, 9H). LCMS: M + H = 384. Under nitrogen protection, (E)-N,N'-bis(3-ethynyl-2-fluorophenyl)fomamidine (390 g, 1.1 equivalents) and glacial acetic acid (1 V) were added dropwise to a toluene solution (8 V) of (R)-4-(4-amino-5-cyano-2-methoxyphenoxy)-3,3-difluoropiperidine-1-carboxylic acid tert-butyl ester (486 g, 1 equivalent) at room temperature, heated to 95 ± 5°C and refluxed for 2 h until the raw material disappeared, cooled to 20 ± 5°C, continued to stir for 12 h, and filtered to obtain (R)-4-(4-anlino-5-cyano-2-methoxyphenoxy)-3,3-difluoropiperidine-1-carboxylic acid tert-butyl ester (540 g, yield > 80%).¹HNMR: (400 MHz, DMSO-d6) δppm: 9.54 (s, 1H), 8.41 (s, 1H), 8.04 (s, 1H), 7.69 - 7.57 (m, 1H), 7.34 - 7.23 (m, 2H), 4.95 - 4.89 (m, 1H), 4.56 (s, 1H) ,3.98 (s, 3H), 3.92 - 3.75 (m, 2H), 3.63 - 3.56 (m, 1H), 3.53 - 3.45 (m, 1H), 2.11 - 2.02 (m, 1H), 1.98 - 1.87 (m, 1H), 1.44 (s, 9H)_{∘} LCMS: M + H =529.

In the present disclosure, trans-N,N'-diphenylimine amide is used as a substrate to increase the yield from 40 - 50% to 70 - 80% compared to the original scheme (CN108069946B) in acid catalysis ring closure, reduce material loss and production cost, and provide a large batch of drugs with high quality and controllable impurities, (R)-6-((3,3-difluoro-1-methylpiperidin-4-yl)oxy)-N-(3-ethynyl-2-fluorophenyl)-7-methoxyquinazoline-4-amine, for ongoing clinical trials (NCT04197934) and commercial purposes.

### Embodiment 3 Process Route for Preparation of (R)-6-((3,3-difluoro-1-methylpiperidin-4-yl)oxy)-N-(3-ethynyl-2-fluorophenyl)-7-methoxyquinazoline-4-amine Using Techniques of the Present Disclosure

The process route is as follows:

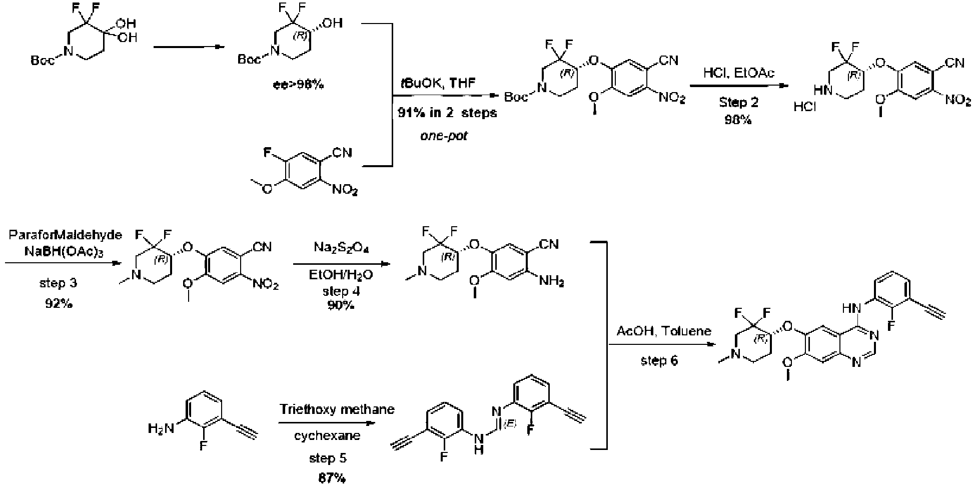

Step I: 3,3-difluoro-4,4-2-hydroxypiperidine-1-carboxylic acid tert-butyl ester (5 kg, 1 equivalent), Ru(II) catalyst RuCl[(R,R-TsDPEN)](p-cymene) (135 g, 1% equivalent) and tetrahydrofuran solution (16.2 L, 5 V) were added to a nitrogen blanketed reactor and stirred until completely dissolved. Triethylamine (4.3 kg, 2 equivalents) was added at room temperature and formaldehyde (1.27 kg, 1.3 equivalents) was added dropwise, and stirred for 2 h at 35 ± 5°C. TLC showed that the raw material disappeared completely. Potassium tert-butoxide (4.29 kg, 1.8 equivalents) and 5-fluoro-4-methoxy-2-nitrobenzonitrile (1 equivalent) were added at 10 ± 5°C, stirred for 2 h, and cooled to 5 ± 5°C. Water (32.4 L, 10 V) was added, stirred to precipitate solids, filtered and dried to obtain a white solid of chiral pure (R)-4-(5-cyano-2-methoxy-4-nitrophenoxy)-3,3-difluoropiperidine-1-carboxylic acid tert-butyl ester (7.95 kg, yield: 91%, %ee > 98%, one-pot synthesis in two steps). ¹H NMR (300 MHz, CDCl₃) δ 7.86 (s, 1H), 7.39 (s, 1H), 4.66 (d, *J =* 6.0 Hz, 1H), 4.05 (s, 3H), 3.79 - 3.67 (m, 2H), 3.45 (m, 1H), 2.11 (s, 2H), 1.62 (s, 1H), 1. 51(s, 9H).

Step 2: (R)-4-(5-cyano-2-methoxy-4-nitrophenoxy)-3,3-difluoropiperidine-1-carboxylic acid tert-butyl ester (7.95 kg, 1 equivalent) and hydrochloric acid/ethyl acetate solution (4M) were added to a nitrogen blanketed reactor, stirred at 25 ± 5°C for 1 h, filtered, and rinsed with ethyl acetate (4 V) to obtain a white solid product of (R)-5-((3,3-difluoropiperidine-4-yl)oxy)-4-methoxy-2-nitrobenzonitrile (6.5 kg, yield: 98%). ¹H NMR (300 MHz, D2O) δ 7.90 (s, 1H), 7.68 (s, 1H), 5.15 - 5.01 (m, 1H), 3.94 (s, 3H), 3.90 - 3.62 (m, 2H), 3.47 - 3.27 (m, 2H), 2.35 - 2.25 (m, 2H).

Step 3: (R)-5-((3,3-difluoropiperidine-4-yl)oxy)-4-methoxy-2-nitrobenzonitrile (5.4 kg, 1 equivalent) and methanol (5 V) were added to a nitrogen blanketed reactor. 37% formaldehyde aqueous solution (5 V, 20 equivalents) and NaBH(OAc)₃ (9.8 kg, 3 equivalents) were added at room temperature, and stirred for 1 h. 15% aqueous sodium hydroxide solution was added to adjust the pH to 8, stirred for 1 h and filtered to obtain a white solid of (R)-5-((3,3-difluoro-1-methylpiperidin-4-yl)oxy)-4-methoxy-2-nitrobenzonitrile (4.66 kg, yield: 92%). ¹H NMR (300 MHz, CD₃Cl) δ 7.87 (s, 1H), 7.40 (s, 1H), 4.65 - 4.50 (m, 1H), 4.10 (s, 3H), 2.98 - 2.71 (m, 2H), 2.70 - 2.49 (m, 2H), 2.40 (s, 3H), 2.25 - 2.15 (m, 2H).

Step 4: (R)-5-((3,3-difluom-1-methylpiperidin-4-yl)oxy)-4-methoxy-2-nitrobenzonitrile (1.2 kg, 1 equivalent) and aqueous ethanol (4 V) were added to a nitrogen blanketed reactor. Sodium dithionite (2.23 kg, 3.5 equivalents) was added at room temperature and stirred for 2 h, then concentrated hydrochloric acid (6.5 equivalents) was added dropwise and stirred for 2 h. Cooled to 15 ± 5°C. 20% sodium hydroxide aqueous solution was added to adjust the pH to 10 - 12, concentrated and filtered, and washed with heptane to obtain (R)-2-amino-5-((3,3-difluoro-1-methylpiperidin-4-yl)oxy)-4-methoxybenzonitrile (980 g, yield: 90%). ¹H NMR (300 MHz, DMSO-*d*₆) δ 7.05 (s, 1H), 6.42 (s, 1H), 5.78 (s, 2H), 4.30 - 4.23 (m, 1H), 3.76 (s, 3H), 2.90 - 2.78 (m, 1H), 2.59 - 2.50 (m, 1H), 2.46 - 2.23 (m, 4H), 1.92 - 1.87 (m, 2H).

Step 5: 3-ethynyl-2-fluoroaniline (1.4 kg, 1 equivalent) and cyclohexane (2.8 V) were added to a nitrogen blanketed reactor. Triethyl orthoformate (0.77 kg, 0.5 equivalent) was added at room temperature and then glacial acetic acid (5.2% equivalent) was added dropwise. Heated to 50 ± 5°C, stirred until the raw material disappeared, concentrated and washed with cyclohexane to obtain (E)-N,N'-bis(3-ethynyl-2-fluorophenyl)formamidine (1.22 kg, yield: 87%). ¹H NMR (300 MHz, DMSO-*d*₆) δ 9.88 (s, 1H), 8.11 (s, 1H), 7.52 - 6.77 (m, 5H), 4.49 (s, 2H);

Step 6: Under nitrogen protection, (E)-N,N'-bis(3-ethynyl-2-fluomphenyl)formamidine (1.17 kg, 1.1 equivalent) and glacial acetic acid (1 V) were added dropwise to a toluene solution (8 V) of (R)-2-amino-5-((3,3-difluom-1-methylpiperidin-4-yl)oxy)-4-methoxybenzonitrile (1.13 kg, 1 equivalent) at room temperature, heated to 95 ± 5°C and refluxed for 2 h until the raw material disappears, cooled to 20 ± 5°C, continued to stir for 12 h, and filtered to obtain (R)-6-((3,3-difluoro-1-methylpiperidin-4-yl)oxy)-N-(3-ethynyl-2-fluorophenyl)-7-methoxyquinazoline-4-amine (1.28 kg, yield > 76%). 1H NMR (300 MHz, DMSO-d6) δ 9.49 (s, 1H), 8.39 (s, 1H), 7.99 (s, 1H), 7.62 (d, J = 7.2 Hz, 1H), 7.45 (d, J = 6.6 Hz, 1H), 7.29 - 7.24 (m, 2H), 4.82 - 4.72 (m, 1H), 4.52 (s, 1H), 3.96 (s, 3H), 2.98 - 2.86 (m, 1H), 2.75 - 2.59 (m, 2H), 2.49 - 2.35 (m, 1H), 2.28 (s, 3H), 2.15 - 1.95 (m, 2H); m/z 443 (100, M+H).

### Embodiment 4 Biological Activity of (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

Cell viability of tumor cells and gliomas with EGFR-activated mutant (Exon 19 Del, PC-9 cell; Exon 21 L858R, H3255 cell) protein was detected using CellTiter-Glo (CTG) assay; approximately 5000 cells were placed in each blank of the 96-well plate. The diluted compound was added proportionally after 16 h and equilibrated at room temperature for 30 min after 72 h of drug addition. 100 µL of CellTiter-Glo reagent was added to each well and mixed well on an orbital shaker for 2 min to induce cell lysis. The plate was incubated at room temperature for 10 min to stabilize the luminous signal. The luminous signal was recorded on a TECAN Infinite M1000 Pro instrument.

**Table 2. Growth Inhibition of Non-small Cell Lung Cancer PC-9, H3255 Tumor Cells and Glioma Tumor Cells (IC₅₀ in nM)**

| | PC-9 tumor cell (EGFR Del 19 mutation) | H3255 tumor cell (EGFR L858R mutation) | Human glioma tumor cell with EGFRVIII mutation |
|---|---|---|---|
| Biological activity of (R)-6-((3,3-difluoro-1-methylpiperidin-4-yl)oxy)-N-(3-ethynyl-2-fluorophenyl)-7-methoxyquinazoline-4-amine | 8nM | 2nM | 10nM |
| EGFR molecules for non-small cell lung cancer, e.g., Erlotinib and Iressa | 40nM | 14nM | >200nM |

Table 2 shows that (R)-6-((3,3-difluoro-1-methylpiperidin-4-yl)oxy)-N-(3-ethynyl-2-fluorophenyl)-7-methoxyquinazoline-4-amine prepared by the present disclosure has unexpected EGFRVIII mutation activity. It has similar biological activity to EGFR Del19, L858R activation mutation. It has higher biological activity than Erlotinib and Iressa.

### Embodiment 5 Significant Improvement in Survival in Animal Models of Intracranial Meningeal Metastases in Mice with (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

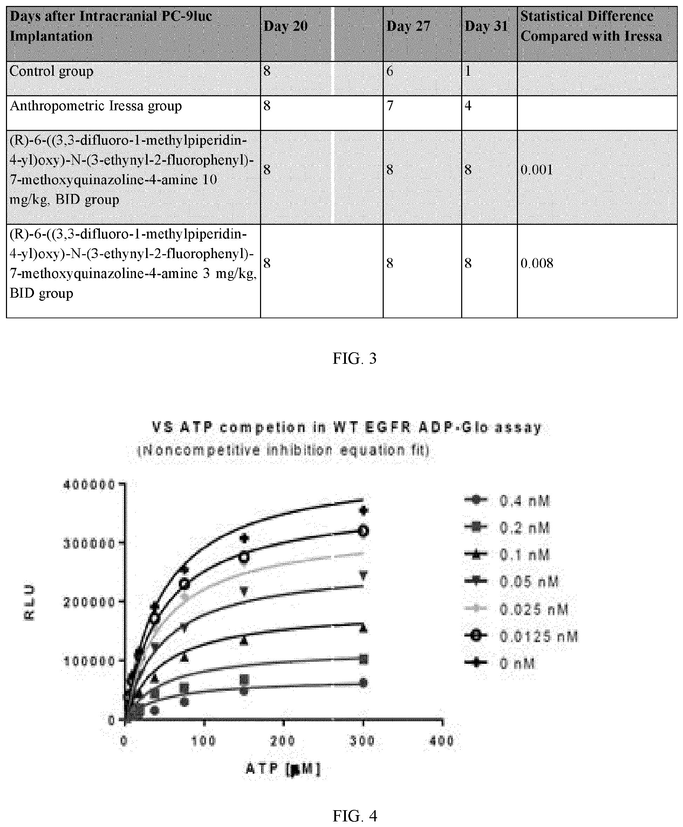

PC-9luc tumor cells (non-small cell lung cancer with Exon 19 Del, about 2 × 10⁵ cells) were seeded into the brain of mice, and administration was started approximately 11 days after tumor growth. Group 1 was the control group (8 mice), without any medication. Group 2 received Iressa (8 mice), 15 mg/kg, PO. Group 3 received (R)-6-((3,3-difluoro-1-methylpiperidin-4-yl)oxy)-N-(3-ethynyl-2-fluorophenyl)-7-methoxyquinazoline-4-amine monotherapy group (8 mice), 10 mg/kg, PO, BID. Group 4 received (R)-6-((3,3-difluoro-1-methylpiperidin-4-yl)oxy)-N-(3-ethynyl-2-fluorophenyl)7-methoxyquinazoline-4-amine monotherapy group (8 mice), 3 mg/kg, PO, BID. As shown in FIG. 3, the survival is significantly improved in Groups 3 and 4, indicating a statistically significant efficacy.

### Embodiment 6 Significant Prolongation of Survival in Animal Models of Intracranial PDX in Mice with (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

The patient's primary tumor tissue GBM (brain tumor, about 50,000 cells) was seeded into the brain of mice. Administration began after the tumor grew for about 10 d and treatment lasted for more than 50 d. In the pharmacodynamic study of tumor transplanted animal model with EGFRV3 mutated brain tumor from patient's primary tumor tissue in mice, Group 1 was the control group (8 mice) without any medication. Group 2 received (R)-6-((3,3-difluoro-1-methylpiperidin-4-yl)oxy)-N-(3-ethynyl-2-fluorophenyl)-7-methoxyquinazoline-4-amine monotherapy group (8 mice), 30 mg/kg, PO, BID. As shown in FIG. 2, the survival of Group 2 was significantly prolonged compared to Group 1 (control group), indicating a statistically significant efficacy.

### Embodiment 7 Rate of Crossing Blood-brain Barrier of (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

To detemmine whether (R)-6-((3,3-difluoro-1-methylpiperidin-4-yl)oxy)-N-(3-ethynyl-2-fluorophenyl)-7-methoxyquinazoline-4-amine prepared by the present disclosure is able to cross the blood-brain barrier (BBB), the test compound was administered to rats. After 4 h of administration, the rats were killed and blood and brain tissues were collected to test the compound concentration. Brain osmosis is defined as the ratio of the concentration of compounds in brain tissue to the concentration in plasma. The rate of crossing blood-brain barrier is the ratio of free drug concentration in brain tissue to free drug concentration in plasma. P-glycoprotein is an efflux protein at the blood-brain barrier that excretes P-glycoprotein substrate from the brain. Breast cancer resistance protein is an efflux protein at the blood-brain barrier that excretes breast cancer resistance protein from the brain.

**Table 3. Ability of (R)-6-((3,3-difluoro-1-methylpiperidin-4-yl)oxy)-N-(3-ethynyl-2-fluorophenyl)-7-methoxyquinazoline-4-amine to Cross the Blood-Brain Barrier**

| | (R)-6-((3,3-difluoro-1-methylpiperidin-4-yl)oxy)-N-(3-ethynyl-2-fluorophenyl)-7-methoxyquinazoline-4-amine |
|---|---|
| Rate of crossing blood-brain barrier | >50% |
| P-glycoprotein efflux substrate | No |
| Breast cancer resistance protein efflux substrate | No |

In the testing of rate of crossing blood-brain barrier, (R)-6-((3,3-difluoro-1-methylpiperidin-4-yl)oxy)-N-(3-ethynyl-2-fluorophenyl)-7-methoxyquinazoline-4-amine has a ratio of free compound concentration in brain tissue to free compound concentration in plasma greater than 50% and is not P-glycoprotein substrate or breast cancer resistance protein efflux substrate that can cross the blood-brain barrier. Therefore, it has the potential to achieve effective plasma concentration intracranially for the treatment of gliomas or for the treatment and prevention of cancerous brain metastases, meningeal metastases, brain cancer and other central nervous system diseases, and reduce the risk of dose-limiting toxicity occurring extracranially.

### Embodiment 8 Good Bioavailability of (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

In the pharmacokinetic study of rats, Group 1 (3 rats) was administrated with 1 - 2 mg/kg, iv, and Group 2 (3 rats) was administrated with 2 - 40 mg/kg, PO. Blood was sampled at 7 time points (0.25, 0.5, 1, 2, 4, 8 and 16 hours points) to measure the concentration of the quinazoline derivatives (I) prepared by the present disclosure in blood and calculate the peak area and half-life. Bioavailability is calculated as (peak area of oral drug/oral dose)/(peak area of intravenous drug/intravenous dose) x 100%. It has good bioavailability, > 35%.

### Embodiment 9 Mechanism of Action of (R)-6-((3,3-difluoro-1-methylpiperidin-4-yl)oxy)-N-(3-ethynyl-2-fluorophenyl)-7-methoxyquinazoline-4-amine Prepared Using Techniques of the Present Disclosure

In the study of the mechanism of action of (R)-6-((3,3-difluoro-1-methylpiperidin-4-yl)oxy)-N-(3-ethynyl-2-fluorophenyl)-7-methoxyquinazoline-4-amine prepared by the present disclosure to inhibit EGFR, EGFR enzyme was added to a solution of PolyE4Y1 on a 384-well plate from 6 concentrations diluted at an equal ratio of 0.4 nM (2-fold) to 0.0125 nM and 0 concentration, and incubated for 15 min. ATP was added at 7 concentrations diluted at an equal ratio of 600 uM (2-fold) for reaction for 60 min, and then ADP-Glo reagent and detection reagent were added for 40 min to measure RLU signal. As shown in FIG. 4, said (R)-6-((3,3-difluoro-1-methylpiperidin-4-yl)oxy)-N-(3-ethynyl-2-fluorophenyl)-7-methoxyquinazoline-4-amine of the present disclosure shows an unexpected non-ATP competitive mechanism of action. The benefits of this mechanism of action are obvious, with minimal side effects and anti-drug resistance.

To sum up, said preparation method of 6-substituted chiral pure difluoropiperidine quinazoline derivatives described in the present disclosure is used to produce quinazoline derivatives, especially (R)-6-((3,3-difluoro-1-methylpiperidin-4-yl)oxy)-N-(3-ethynyl-2-fluorophenyl)-7-methoxyquinazoline-4-amine with chirality of R, due to its high activity, high selectivity, high rate of crossing blood-brain barrier, non-efflux substrate, inhibitory property of non-ATP competition mechanism, significant pharmacodynamic effect on non-small cell lung cancer and brain glioma, and high bioavailability. It has great potential to be applied in the treatment of non-small cell lung cancers with brain metastases, meningeal metastases, squamous cell carcinoma of the head and neck, squamous cell carcinoma, brainstem tumors, primary brain cancers, lung squamous cell carcinoma or glioma. The present disclosure has developed a drug manufacturing process with high yield, high chiral selectivity, optimized repeatability, safety and impurity controllability, which is of great significance for scale-up clinical and commercial purposes and reduction of production costs.

## Claims

1. A preparation method for 6-substituted chiral pure difluoropiperidine quinazoline derivative, wherein the method comprises a step of quinazoline ring closing: 2-amino-benzonitrile derivative 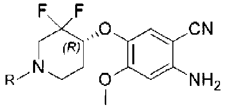 reacts with (E)-N,N'-bisphenylformamidine derivative 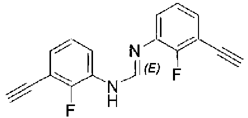 in the presence of organic solvent and glacial acetic acid to obtain 6-substituted chiral pure difluoropiperidine quinazoline derivative 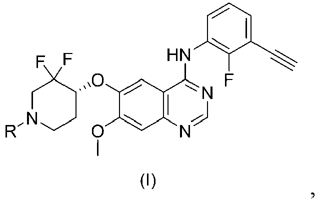 where R is Boc, oxetane, or methyl;
wherein the preparation of said 2-amino-benzonitrile derivative comprises the following steps:
S1: The substituted chiral difluoropiperidinol 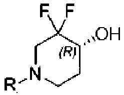 reacts with 5-fluoro-4-methoxy-2-nitrobenzonitrile 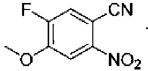 in the presence of potassium tert-butoxide and an organic solvent at 10± 5°C to obtain a 2-nitro-benzonitrile derivative 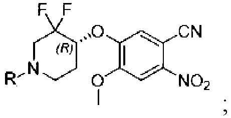
S2: Under nitrogen protection, the 2-nitro-benzonitrile derivative 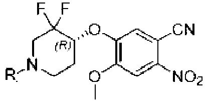 is reduced with sodium dithionite and an organic solvent at room temperature to obtain a 2-amino-benzonitrile derivative

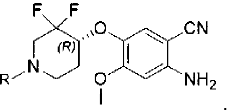

2. The preparation method for 6-substituted chiral pure difluoropiperidine quinazoline derivative according to claim 1, wherein the said 2-amino-benzonitrile derivative is (R)-4-(4-amino-5-cyano-2-methoxyphenoxy)-3,3-difluoropiperidine-1-carboxylic acid tert-butyl ester.

3. The preparation method for 6-substituted chiral pure difluoropiperidine quinazoline derivative according to claim 1, wherein the organic solvent is toluene; the reaction temperature is 90-110°C and the reaction time is 2-4 h; the equivalent ratio of the 2-amino-benzonitrile derivative to (E)-N,N'-bisphenylformamidine derivative is 1:(1-1.2).

4. The preparation method for 6-substituted chiral pure difluoropiperidine quinazoline derivative according to claim 1, wherein when the structural formula of 2-nitro-benzonitrile derivative in step S1 is 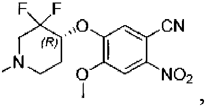 step S1 comprises:
S1-1: Substituted chiral difluoropiperidinol 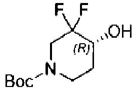 reacts with 5-fluoro-4-methoxy-2-nitrobenzonitrile 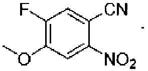 in the presence of potassium tert-butoxide and organic solvent to obtain chiral pure (R)-4-(5-cyano-2-methoxy-4-nitrophenoxy)-3,3-difluoropiperidine-1-carboxylic acid tert-butyl ester 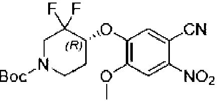 at 10±5°C;
S1-2: Chiral pure (R)-4-(5-cyano-2-methoxy-4-nitrophenoxy)-3,3-difluoropiperidine-1-carboxylic acid tert-butyl ester reacts in the presence of hydrochloric acid/ethyl acetate solution at 25±5°C to obtain (R)-5-((3,3-difluoropiperidine-4-yl)oxy)-4-methoxy-2-nitrobenzonitrile 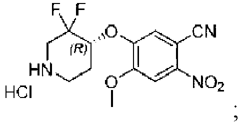
S1-3: Under nitrogen protection, (R)-5-((3,3-difluoropiperidine-4-yl)oxy)-4-methoxy-2-nitrobenzonitrile reacts in the presence of an organic solvent, formaldehyde aqueous solution, and NaBH(OAc)₃ to generate 2-nitro-benzonitrile derivative

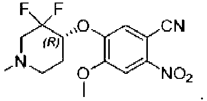

5. The preparation method for 6-substituted chiral pure difluoropiperidine quinazoline derivative according to claim 1, wherein the preparation of said substituted chiral difluoropiperidinol comprises the following steps: Mix the 3,3-difluoropiperidinol derivative 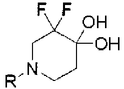 with triethylamine and formaldehyde in the presence of chiral Ru(II) catalyst and organic solvent for reaction until TLC shows that the raw materials disappear to obtain substituted chiral difluoropiperidinol

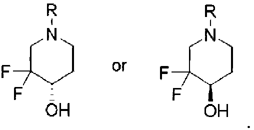

6. The preparation method for 6-substituted chiral pure difluoropiperidine quinazoline derivative according to claim 5, wherein the 3,3-difluoropiperidinol derivative is 3,3-difluoro-1-methylpiperidin-4-keto or 3,3-difluoro-4-oxopiperidin-1-carboxylic acid tert-butyl ester.

7. The preparation method for 6-substituted chiral pure difluoropiperidine quinazoline derivative according to claim 5, wherein the said chiral Ru(II) catalyst is (*R*)-RuCl[(p-cymene(BINAP)]Cl or RuCl[(R,R-TsDPEN)](p-cymene), and 3,3-difluoropiperidinol derivative 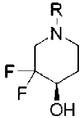 with chirality R is obtained.

8. The preparation method for 6-substituted chiral pure difluoropiperidine quinazoline derivative according to claim 5, wherein the said chiral Ru(II) catalyst is (S)-RuCl[(p-cymene(BINAP)]Cl or RuCl[(S,S-TsDPEN)](p-cymene) and 3,3-difluoropiperidinol derivative 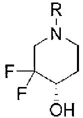 with chirality S is obtained.

9. The preparation method for 6-substituted chiral pure difluoropiperidine quinazoline derivative according to claim 5, wherein the said organic solvent is DMF, DME, or tetrahydrofuran; the said base is potassium tert-butoxide; and the equivalent ratio of the said 3,3-difluoropiperidinol derivative, triethylamine, formaldehyde, and base is 1:(1.5-2):(1-1.5):(1-2).

## Patentansprüche

1. Herstellungsverfahren für 6-substituiertes chirales reines Difluorpiperidinchinazolinderivat, wobei das Verfahren einen folgenden Schritt des Chinazolinringschließens umfasst: 2-Aminobenzonitrilderivat 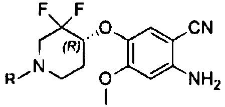 reagiert mit (E)-N,N'-Bisphenylformamidinderivat 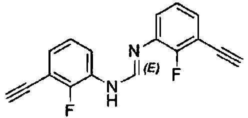 in der Gegenwart von organischem Lösungsmittel und Eisessigsäure, um 6-substituiertes chirales reines Difluorpiperidinchinazolinderivat 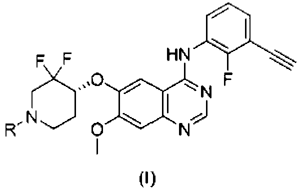 zu erhalten, wobei R Boc, Oxetan oder Methyl ist;
wobei die Herstellung des 2-Aminobenzonitrilderivats die folgenden Schritte umfasst:
S1: Das substituierte chirale Difluorpiperidinol 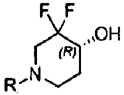 reagiert mit 5-Fluor-4-methoxy-2-nitrobenzonitril 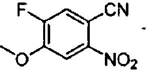 in der Gegenwart von Kalium-tert-butoxid und einem organischen Lösungsmittel bei 10±5 °C, um ein 2-Nitrobenzonitrilderivat 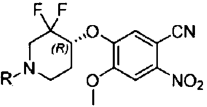 zu erhalten;
S2: Unter Stickstoffschutz wird das 2-Nitrobenzonitrilderivat 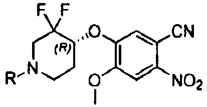 mit Natriumdithionit und einem organischen Lösungsmittel bei Raumtemperatur reduziert, um ein 2-Aminobenzonitrilderivat 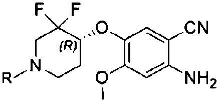 zu erhalten.

2. Herstellungsverfahren für 6-substituiertes chirales reines Difluorpiperidinchinazolinderivat nach Anspruch 1, wobei das 2-Aminobenzonitrilderivat (R)-4-(4-Amino-5-cyano-2-methoxyphenoxy)-3,3-difluorpiperidin-1-carbonsäure-tert-butylester ist.

3. Herstellungsverfahren für 6-substituiertes chirales reines Difluorpiperidinchinazolinderivat nach Anspruch 1, wobei das organische Lösungsmittel Toluol ist; die Reaktionstemperatur 90-110 °C ist und die Reaktionszeit 2-4 h ist; das Äquivalentverhältnis des 2-Aminobenzonitrilderivats zu (E)-N,N'-Bisphenylformamidinderivat 1:(1-1,2) ist.

4. Herstellungsverfahren für 6-substituiertes chirales reines Difluorpiperidinchinazolinderivat nach Anspruch 1, wobei, wenn die Strukturformel von 2-Nitrobenzonitrilderivat in Schritt S1 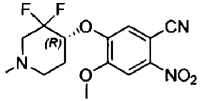 ist, Schritt S1 Folgendes umfasst:
S1-1: Substituiertes chirales Difluorpiperidinol 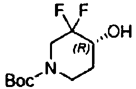 reagiert mit 5-Fluor-4-methoxy-2-nitrobenzonitril 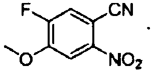 in der Gegenwart von Kalium-tert-butoxid und organischem Lösungsmittel, um chiralen reinen (R)-4-(5-Cyano-2-methoxy-4-nitrophenoxy)-3,3-difluorpiperidin-1-carbonsäure-tert-butylester 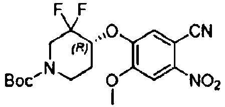 bei 10±5 °C zu erhalten;
S1-2: Chiraler reiner (R)-4-(5-Cyano-2-methoxy-4-nitrophenoxy)-3,3-difluorpiperidin-1-carbonsäure-tert-butylester reagiert in der Gegenwart von Salzsäure/Ethylacetat-Lösung bei 25±5 °C, um (R)-5-((3,3-Difluorpiperidin-4-yl)oxy)-4-methoxy-2-nitrobenzonitril 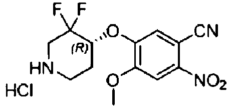 zu erhalten;
S1-3: Unter Stickstoffschutz reagiert (R)-5-((3,3-Difluorpiperidin-4-yl)oxy)-4-methoxy-2-nitrobenzonitril in der Gegenwart eines organischen Lösungsmittels, wässriger Formaldehydlösung und NaBH(OAc)₃, um 2-Nitrobenzonitrilderivat 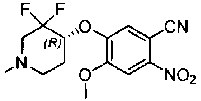 zu erzeugen.

5. Herstellungsverfahren für 6-substituiertes chirales reines Difluorpiperidinchinazolinderivat nach Anspruch 1, wobei die Herstellung des substituierten chiralen Difluorpiperidinols die folgenden Schritte umfasst: Mischen des 3,3-Difluorpiperidinolderivats 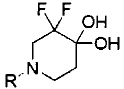 mit Triethylamin und Formaldehyd in der Gegenwart von chiralem Ru(II)-Katalysator und organischem Lösungsmittel zur Reaktion, bis TLC zeigt, dass die Rohstoffe verschwinden, um substituiertes chirales Difluorpiperidinol 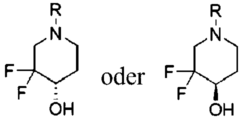 zu erhalten.

6. Herstellungsverfahren für 6-substituiertes chirales reines Difluorpiperidinchinazolinderivat nach Anspruch 5, wobei das 3,3-Difluorpiperidinolderivat 3,3-Difluor-1-methylpiperidin-4-keto oder 3,3-Difluor-4-oxopiperidin-1-carbonsäure-tert-butylester ist.

7. Herstellungsverfahren für 6-substituiertes chirales reines Difluorpiperidinchinazolinderivat nach Anspruch 5, wobei der chirale Ru(II)-Katalysator (*R*)-RuCl[(p-Cymol(BINAP)]Cl oder RuCl[(R,R-TsDPEN)](p-Cymol) ist und 3,3-Difluorpiperidinolderivat 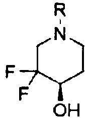 mit Chiralität R erhalten wird.

8. Herstellungsverfahren für 6-substituiertes chirales reines Difluorpiperidinchinazolinderivat nach Anspruch 5, wobei der chirale Ru(II)-Katalysator (S)-RuCl[(p-Cymol(BINAP)]Cl oder RuCl[(S,S-TsDPEN)](p-Cymol) ist und 3,3-Difluorpiperidinolderivat 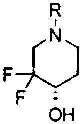 mit Chiralität S erhalten wird.

9. Herstellungsverfahren für 6-substituiertes chirales reines Difluorpiperidinchinazolinderivat nach Anspruch 5, wobei das organische Lösungsmittel DMF, DME oder Tetrahydrofuran ist; die Base Kalium-tert-butoxid ist; und das Äquivalentverhältnis des 3,3-Difluorpiperidinolderivats, Triethylamins, Formaldehyds und der Base 1:(1,5-2):(1-1,5):(1-2) ist.

## Revendications

1. Procédé de préparation d'un dérivé chiral pur de difluoropipéridine quinazoline substitué en position 6, ledit procédé comprenant une étape de fermeture de cycle quinazoline : un dérivé 2-amino-benzonitrile 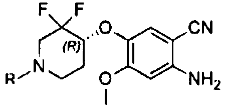 réagit avec un dérivé (E)-N,N'-bisphénylformamidine 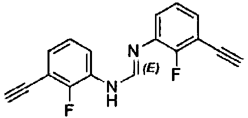 en présence de solvant organique et d'acide acétique glacial pour obtenir un dérivé chiral pur de difluoropipéridine quinazoline substitué en position 6 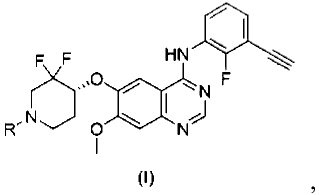
où R est Boc, un oxétane ou un méthyle ;
ladite préparation dudit dérivé 2-amino-benzonitrile comprenant les étapes suivantes :
S1 : le difluoropipéridinol chiral substitué 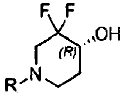 réagit avec le 5-fluoro-4-méthoxy-2-nitrobenzonitrile 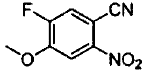 en présence de tert-butoxyde de potassium et d'un solvant organique à 10 ± 5 °C pour obtenir un dérivé 2-nitro-benzonitrile 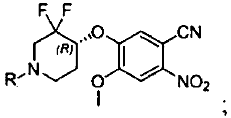
S2 : sous protection à l'azote, le dérivé 2-nitro-benzonitrile 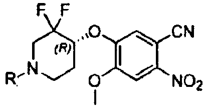 est réduit avec du dithionite de sodium et un solvant organique à température ambiante pour obtenir un dérivé 2-aminobenzonitrile

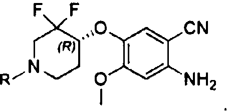

2. Procédé de préparation d'un dérivé chiral pur de difluoropipéridine quinazoline substitué en position 6 selon la revendication 1, dans lequel ledit dérivé 2-amino-benzonitrile est l'ester tert-butylique de l'acide (R)-4-(4-amino-5-cyano-2-méthoxyphénoxy)-3,3-difluoropipéridine-1-carboxylique.

3. Procédé de préparation d'un dérivé chiral pur de difluoropipéridine quinazoline substitué en position 6 selon la revendication 1, dans lequel le solvant organique est le toluène ; la température de réaction est de 90 à 110 °C et le temps de réaction est de 2 à 4 h ; le rapport équivalent du dérivé 2-amino-benzonitrile au dérivé (E)-N,N'-bisphénylformamidine est de 1:(1-1,2).

4. Procédé de préparation d'un dérivé chiral pur de difluoropipéridine quinazoline substitué en position 6 selon la revendication 1, dans lequel lorsque la formule structurale du dérivé 2-nitro-benzonitrile à l'étape S1 est 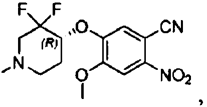 l'étape S1 comprend :
S1-1 : le difluoropipéridinol chiral substitué 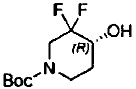 réagit avec le 5-fluoro-4-méthoxy-2-nitrobenzonitrile 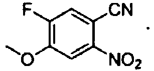 en présence de tert-butoxyde de potassium et de solvant organique pour obtenir l'ester tert-butylique de l'acide (R)-4-(5-cyano-2-méthoxy-4-nitrophénoxy)-3,3-difluoropipéridine-1-carboxylique pur chiral 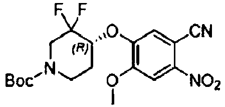 à 10 ± 5°C ;
S1-2: l'ester tert-butylique de l'acide (R)-4-(5-cyano-2-méthoxy-4-nitrophénoxy)-3,3-difluoropipéridine-1-carboxylique pur chiral réagit en présence d'une solution d'acide chlorhydrique/acétate d'éthyle à 25 ± 5 °C pour obtenir le (R)-5-((3,3-difluoropipéridine-4-yl)oxy)-4-méthoxy-2-nitrobenzonitrile 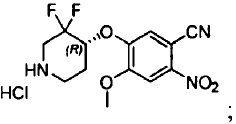
S1-3: sous protection à l'azote, le (R)-5-((3,3-difluoropipéridine-4-yl)oxy)-4-méthoxy-2-nitrobenzonitrile réagit en présence d'un solvant organique, d'une solution aqueuse de formaldéhyde et de NaBH(OAc)₃ pour générer un dérivé 2-nitro-benzonitrile

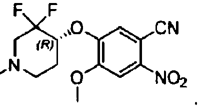

5. Procédé de préparation d'un dérivé chiral pur de difluoropipéridine quinazoline substitué en position 6 selon la revendication 1, dans lequel la préparation dudit difluoropipéridinol chiral substitué comprend les étapes suivantes : le mélange du dérivé 3,3-difluoropipéridinol 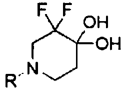 avec la triéthylamine et le formaldéhyde en présence d'un catalyseur chiral Ru(II) et d'un solvant organique pour la réaction jusqu'à ce que la CCM montre la disparition des matières premières pour obtenir le difluoropipéridinol chiral substitué

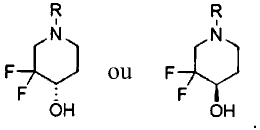

6. Procédé de préparation d'un dérivé chiral pur de difluoropipéridine quinazoline substitué en position 6 selon la revendication 5, dans lequel le dérivé 3,3-difluoropipéridinol est l'ester tert-butylique de l'acide 3,3-difluoro-1-méthylpipéridine-4-céto ou 3,3-difluoro-4-oxopipéridine-1-carboxylique.

7. Procédé de préparation d'un dérivé chiral pur de difluoropipéridine quinazoline substitué en position 6 selon la revendication 5, dans lequel ledit catalyseur chiral Ru(II) est (*R*)-RuCl[(p-cymène(BINAP)]Cl ou RuCl[(R,R-TsDPEN)](p-cymène), et un dérivé 3,3-difluoropipéridinol 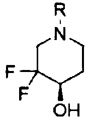 présentant une chiralité R est obtenu.

8. Procédé de préparation d'un dérivé chiral pur de difluoropipéridine quinazoline substitué en position 6 selon la revendication 5, dans lequel ledit catalyseur chiral Ru(II) est (S)-RuCl[(p-cymène(BINAP)]Cl ou RuCl[(S,S-TsDPEN)](p-cymène) et un dérivé 3,3-difluoropipéridinol 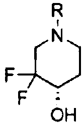 présentant une chiralité S est obtenu.

9. Procédé de préparation d'un dérivé chiral pur de difluoropipéridine quinazoline substitué en position 6 selon la revendication 5, dans lequel ledit solvant organique est le DMF, le DME ou le tétrahydrofurane ; ladite base est le tert-butoxyde de potassium ; et le rapport équivalent dudit dérivé 3,3-difluoropipéridinol, de ladite triéthylamine, dudit formaldéhyde et de ladite base est de 1:(1,5-2):(1-1,5):(1-2).
